# EUROPÄISCHE PATENTSCHRIFT

(11) **EP 1 762 193 B1**
(45) Veröffentlichungstag und Bekanntmachung des Hinweises auf die Patenterteilung: **22.04.2009**
(21) Anmeldenummer: 06018543.6
(22) Anmeldetag: 05.09.2006
(51) Int. Cl.: A61B 17/28, A61B 17/32, A61B 19/00

(54) **Abstandhalter für ein medizinisches Instrument**
Spacer for a medical instrument
Entretoise pour un instrument médical

(30) Priorität: 09.09.2005 DE 102005044364
(43) Veröffentlichungstag der Anmeldung: 14.03.2007
(73) Patentinhaber: Karl Storz GmbH & Co. KG, 78532 Tuttlingen (DE)
(72) Erfinder: Wallwiener, Diethelm, Prof. Dr., 72076 Tübingen (DE); Brucker, Sara, Dr., 72810 Gomaringen/Stockach (DE)
(74) Vertreter: Weller, Wolfgang

(56) Entgegenhaltungen:
- EP-A- 1 029 509
- WO-A-01/06966
- DE-A1- 3 048 758
- DE-U1- 8 625 288
- DE-U1- 20 209 525
- US-A- 4 759 363
- US-A1- 2002 111 563
- US-B1- 6 716 215

## Beschreibung

Die Erfindung betrifft einen Abstandhalter zur Begrenzung der Einschubtiefe eines Schaftes eines medizinischen Instrumentes in einen Körper, mit einem distalen Anschlag zum Anlegen am Körper, mit einem sich über einen Längenabschnitt des Schaftes erstreckenden Abstandselement, wobei der Abstandhalter lösbar am medizinischen Instrument anbringbar ist, und mit einer Öffnung, über die der Abstandhalter am Schaft anbringbar ist.

Ein derartiger Abstandhalter ist aus der DE 30 48 758 A1 bekannt. Der Abstandhalter besteht aus einem U-förmig gebogenen Blechstreifen, der seitlich an den Schaft einer medizinischen Fasszange anklipsbar ist und daher als Reiter bezeichnet wird. An dem Reiter ist ein Halteansatz vorgesehen, der einmal zur leichteren Betätigung des Reiters beim Einstellen des gewünschten Abstandes von der Zangenspitze und zum anderen als Markierungshilfe dient. Dieser Halteansatz dient auch als Anschlag beim Einführen des medizinischen Instrumentes.

Aus der DE 86 25 288 U1 ist eine geschlitzte Anschlaghülse mit einem Innendurchmesser von 1,6-4,5 mm und einer Länge von 1,5-6,5 mm bekannt, die an einem Ende ein stufenförmiges Ende oder eine größere Wandstärke aufweist, die zur Bestimmung der Bohr- und Frästiefe in einem Kieferknochen dient.

Im Rahmen der minimal-invasiven Chirurgie haben sich medizinische Instrumente etabliert, die über eine kleine Inzision in den Körper einführbar sind, um im Innern des Körpers einen Eingriff vorzunehmen.

Die medizinischen Instrumente weisen einen Schaft auf, der über die Inzision in den Körper eingeführt wird, beispielsweise durch die Bauchdecke hindurch in die Bauchhöle. Es können auch natürliche Körperöffnungen wie der Vaginal- oder Analbereich herangezogen werden, um derartige Instrumente einzuführen. Der Schaft selber kann massiv ausgebildet sein, z. B. bei einem Leberhaken, einem Trokardorn oder sonstigen Manipulationsinstrumenten, oder er kann als hohler Schaft ausgebildet sein.

Durch den Hohlschaft hindurch können verschiedenartige Instrumente für Eingriffe durchgeführt werden.

Proximalseitig endet der Schaft meist in einem durchmessergrößeren Bauelement, beispielsweise einem Gehäuse, einem Griff oder dgl.

Der Schaft weist eine bestimmte Länge auf und kann theoretisch so weit in den Körper eingeschoben werden, bis das Instrument über das proximalseitige durchmessergrößere Bauteil am Körper im Bereich um die Öffnung, sei es eine Inzision, sei es eine natürliche Öffnung, zum Liegen kommt.

Sind während einer Operation durch einen hohlen Schaft Instrumente hindurchgeschoben, die an einer Stelle im Körper z.B. einen Gewebe abtragenden Vorgang durchführen sollen, so variiert von Patient zu Patient der Abstand dieser Stelle von der Außenseite des Körpers, die die maximale Einschubtiefe des Instrumentes begrenzt.

Man kann zwar durch die Wahl der Länge des Schaftes gewisse maximale Einschubtiefen begrenzen, je nach Art der Operation, der anatomischen Gegebenheiten und insbesondere auch der Größe und der Statur des Patienten ist aber dieser Abstand eine variable Größe.

Es muss der Operateur daher eine erhöhte Aufmerksamkeit dahingehend aufwenden, dass der Schaft eine bestimmte Einschubtiefe nicht überschreitet.

Ein Beispiel ist die Einschubtiefe der Trokarhülse beim Setzen eines Trokars. Trokare bestehen üblicherweise aus einem Hohlschaft, der proximalseitig über ein durchmessergrößeres Ventilgehäuse abgeschlossen ist. Zum Setzen des Trokars im Körper wird in die Trokarhülse ein angespitzter Trokardorn eingeschoben, dessen scharfe Spitze die Trokarhülse, also den Schaft, distalseitig überragt. Diese Trokardornspitze wird an eine zuvor am Körper angebrachten Inzision angesetzt, beispielsweise an der Bauchdecke, und durch eine kräftige Schubbewegung wird der Zusammenbau aus Trokardorn und Trokarhülse durch die Bauchdecke in die Bauchhöhle eingetrieben. Nach diesem Vorgang wird der Trokardorn abgezogen. Dabei kann die Trokarhülse theoretisch so weit in die Bauchhöhle eingetrieben werden, bis das Ventilgehäuse an der Bauchdecke zum Liegen kommt. Dabei kann nicht ausgeschlossen werden, dass beim Eintreiben der Trokarhülse der distalseitig vorstehende Trokardorn Verletzungen im Innern des Körpers bewirkt. Wird ein Trokardorn eingesetzt, der wesentlich länger als die Trokarhülse ist, besteht eine erhebliche Verletzungsgefahr, wenn der Trokardorn zu weit eingetrieben wird.

Noch weitreichendere Folgen können verursacht werden, wenn ein medizinisches Instrument eingesetzt wird, durch dessen Schaft Gewebe ablösende Instrumente hindurchgeführt werden. Ein Beispiel für ein derartiges ist ein sog. Morcellator.

Ein derartiger Morcellator ist beispielsweise durch die Anmelderin unter der Bezeichnung "ROTOCUT" in dem Katalog Endoworld Gyn 20-1-E/11-2004 beschrieben. Ein Morcellator weist ein relativ großes durchmesserstarkes Gehäuse auf, in dem ein Motor aufgenommen ist, von dem distalseitig ein Hohlschaft vorsteht, der, ähnlich wie zuvor wie im Zusammenhang mit einem Trokar beschrieben, in einen Körper eingeführt werden kann. In dem Schaft ist ein durch den Motor antreibbares Schneidewerkzeug aufgenommen, das ebenfalls aus einem Rohr besteht und das distalseitig umfänglich mit einer Schneide versehen ist. Durch die rohrförmige Schneide hindurch kann zusätzlich ein Fasswerkzeug hindurchgeschoben werden, um beispielsweise ein Gewebe zu erfassen und zu halten, das durch die Schneide abgetrennt werden soll. Beim eigentlichen Abtragvorgang wird der Morcellator über eine gewisse Strecke vorgetrieben, die der Höhe des abzutragenden Gewebes entspricht, die je nach den anatomischen Gegebenheiten bzw. dem pathologischen Fall unterschiedlich ist. Auch hier hängt es wieder von der Geschicklichkeit des Operateurs ab, wie weit er den Morcellator beim Abtragvorgang in den Körper vorschiebt. Ein zu weites Vorschieben würde verursachen, dass nicht nur das pathologische Gewebe, sondern auch darunter liegendes gesundes Gewebe abgetragen würde, was auf jeden Fall vermieden werden soll.

Ein Abstandhalter soll flexibel einsetzbar sein, d. h. er soll ggf. auch dann herangezogen werden können, wenn das Instrument schon im Körper eingeschoben ist.

Die Aufgabe wird durch einen Abstandhalter gelöst, bei dem der Anschlag als scheibenartiger Körper ausgebildet ist, und die Öffnung als seitliche Schlitzöffnung in dem scheibenförmigen Körper ausgebildet ist, über die der Abstandhalter seitlich an den Schaft anlegbar ist, so dass der Schaft etwa mittig in dem geschlitzten scheibenartigen Körper zum Liegen kommt.

Die Ausgestaltung des Anschlages als scheibenartiger Körper hat den Vorteil, dass das Anhalten des Einschubvorganges den Körper nicht beeinträchtigt, da die Kräfte über den flächigen Körper verteilt werden können. Gleichzeitig kann ausgeschlossen werden, auch wenn mit relativ hoher Kraft die Einschubbewegung des Instrumentes durchgeführt wird, dass durch Eindrücken des Körpergewebes das Instrument doch noch zu weit eingeführt wird. Die Scheibengeometrie leitet diese Kraft sehr sanft ab, bzw. übt einen sehr sanften flächigen Anschlagwiderstand aus.

In einer weiteren Ausgestaltung der Erfindung sind mehrere Abstandhalter aneinander koppelbar.

Diese Maßnahme hat den Vorteil, dass das Maß der Begrenzung der Einschubtiefe durch das Aneinanderkoppeln mehrerer Abstandhalter veränderbar ist. Nimmt man das vorangegangene Beispiel mit dem Abstandhalter mit der seitlichen Öffnung heran, so kann, nachdem ein Abstandhalter seitlich aufgeschoben wurde und die Einschubtiefe weiter begrenzt werden soll, einfach noch ein weiterer zusätzlicher Abstandhalter aufgeschoben werden.

Dies kann entweder so geschehen, dass auf den bereits angelegten Abstandhalter ein weiterer aufgeschoben wird, oder dass dieser kurzfristig abgenommen wird, mit einem zweiten gekoppelt wird und dann der Zusammenbau aus den zwei oder ggf. mehreren Abstandhaltern wieder am Schaft angelegt wird.

In einer weiteren Ausgestaltung der Erfindung weist das Abstandselement einen vom Anschlag abstehenden ersten Ringflansch auf, der über eine Schulter in einen zweiten durchmessergrößeren Ringflansch übergeht.

Diese Ausgestaltung eröffnet nun zum einen die Möglichkeit, mehrere solche Abstandselemente einfach aneinander zu koppeln bzw. übereinander zu stapeln. Zugleich ist durch die entsprechende axiale Längenausdehnung des durchmessergrößeren zweiten Ringflansches es möglich, ganz bestimmte Einschubtiefenreduzierungen pro Abstandselement vorzugeben, beispielsweise in Zentimeterabschnitten oder 2-Zentimeterabschnitten oder dgl.

In einer weiteren Ausgestaltung der Erfindung entspricht der Außendurchmesser des ersten Ringflansches etwa dem lichten Innendurchmesser des zweiten Ringflansches.

Diese Maßnahme hat den Vorteil, dass mehrere solche ausgestaltete Abstandselemente relativ festsitzend aufeinanderaufgeschoben werden können. Mehrere aufeinandergeschobene Abstandselemente ergeben dann einen etwa zylindrischen Grundkörper, der aus den jeweils zweiten durchmessergrößeren Ringflanschen zusammengesetzt ist, lediglich vom obersten Abstandselement des jeweiligen Stapels steht der erste Ringflansch mit etwas geringerem Durchmesser vor, der dann in den eigentlichen Anschlag, nämlich den Scheiben- oder flächigen Körper übergeht.

In einer weiteren Ausgestaltung der Erfindung ist der lichte Innendurchmesser des zweiten Ringflansches so gewählt, dass er auf einen entsprechenden Vorsprung am Instrument ansetzbar ist.

Diese Maßnahme hat nun den erheblichen Vorteil, dass ein Abstandselement festsitzend und in einer bestimmten Position sitzend am Instrument anbringbar ist.

Greift man auf das Beispiel mit der seitlichen Schlitzöffnung zurück, kann der Abstandhalter in irgendeiner Längenposition des Schaftes seitlich aufgeschoben werden und kann durch axiales Verschieben nach proximal so weit verschoben werden, bis der zweite durchmessergrößere Ringflansch auf dem entsprechenden Vorsprung am Instrument aufgesetzt ist. Auf ein solches bereits angesetztes Abstandselement können dann ggf. weitere Abstandselemente entsprechend aufgesetzt werden, oder es kann in gleicher Weise auch ein Zusammenbau aus mehreren aufeinandergesetzten Abstandselementen angesetzt werden. Durch die Wahl einer entsprechenden Bemaßung ist es möglich, dass der zweite durchmessergrößere Ringflansch unter Überwindung eines gewissen Widerstandes auf den Vorsprung aufgeschoben oder aufgeclipst werden kann, so dass das so angesetzte Abstandselement unverlierbar und unverrückbar, jedoch lösbar am Instrument sitzt. Diese Ausgestaltung, also das Zusammenwirken von Vorsprung und zweiten durchmessergroßem Ringflansch, stellt ein Teil der Vorrichtung zum Anbringen des Abstandhalters am Instrument dar.

In einer weiteren Ausgestaltung der Erfindung weist die Vorrichtung zum lösbaren Anbringen eine Feststellschraube auf, über die ein am Schaft des Instruments angelegte Abstandhalter am Schaft feststellbar ist.

Diese Maßnahme hat den Vorteil, dass ein Abstandhalter, nachdem er entsprechend gewünscht positioniert ist, in dieser Position über die Feststellschraube feststellbar ist.

In einer weiteren Ausgestaltung der Erfindung ist die Breite der Schlitzöffnung im Abstandhalter geringfügig geringer als der Außendurchmesser des Schaftes, so dass der Abstandhalter im Klemmsitz auf den Schaft anlegbar ist.

Diese Maßnahme hat den Vorteil, dass durch diese Ausgestaltung der Abstandhalter zwar lösbar, aber dennoch schon in einem gewissen Klemmsitz einlegbar ist.

Je nach weiterer Ausgestaltung der Vorrichtung kann nun der Abstandhalter in diesem Klemmsitz am Schaft verbleiben, sofern dieser ausreichend ist und genügend Widerstand gegen Verschieben entgegenzuwirken oder ggf. noch durch eine Feststellschraube zusätzlich gesichert werden. Ist der Klemmsitz so gewählt, dass der Abstandhalter seitlich zwar auf den Schaft aufgeschoben werden, aber noch axial längs diesem verschoben werden kann, dient diese Ausgestaltung lediglich dazu, den Abstandhalter nun einmal zunächst unverlierbar an den Schaft seitlich anzusetzen und anschließend durch Verschieben beispielsweise bis auf den zuvor erwähnten Vorsprung unverlierbar zu halten.

Dies zeigt die Variabilität der Ausgestaltung der Vorrichtung zum lösbaren Anbringen, so dass durch diese Variabilität und beispielsweise entsprechende Materialauswahl Abstandhalter zur Mehrfachverwendung oder auch nur zum Einmalgebrauch ausgestaltet werden können.

Es versteht sich, dass die vorstehend genannten und die nachstehend noch zu erläuternden Merkmale nicht nur in der jeweils angegebenen Kombination, sondern auch in anderen Kombinationen oder in Alleinstellung einsetzbar sind, ohne den Rahmen der vorliegenden Erfindung zu verlassen.

Die Erfindung wird nachfolgend an Hand zweier ausgewählter Ausführungsbeispiele in Zusammenhang mit den beiliegenden Zeichnungen näher beschrieben und erläutert. Es zeigen:
- Fig. 1: eine Draufsicht auf den distalseitigen Anschlag eines ersten Ausführungsbeispiels eines Abstandhalters,
- Fig. 2: eine Seitenansicht des Abstandhalters von Fig. 1,
- Fig. 3: einen Schnitt längs der Linie III-III von Fig. 1,
- Fig. 4: eine perspektivische Ansicht des Abstandhalters von Fig. 1,
- Fig. 5: eine perspektivische Ansicht von drei aufeinandergestapelten bzw. aufeinandergesetzten Abstandhaltern von Fig. 4,
- Fig. 6: stark schematisiert und teilweise im Schnitt eine Situation, bei der der in Fig. 1-4 gezeigte Abstandhalter an einen Schaft eines medizinischen Instruments in Form eines Morcellators angesetzt werden soll, wobei das medizinische Instrument bereits in einen Körper eingeführt ist,
- Fig. 7: eine der Darstellung von Fig. 6 vergleichbare Situation, wobei der aus Fig. 5 ersichtliche Zusammenbau aus drei übereinandergestapelten Abstandhaltern am Schaft angebracht ist,
- Fig. 8: eine den Fig. 6 und 7 vergleichbare Darstellung eines Morcellators, auf den ein weiteres Ausführungsbeispiel eines Abstandhalters in Form einer geschlitzten Scheibe angebracht ist, und
- Fig. 9: eine Draufsicht auf den in Fig. 8 dargestellten Abstandhalter.

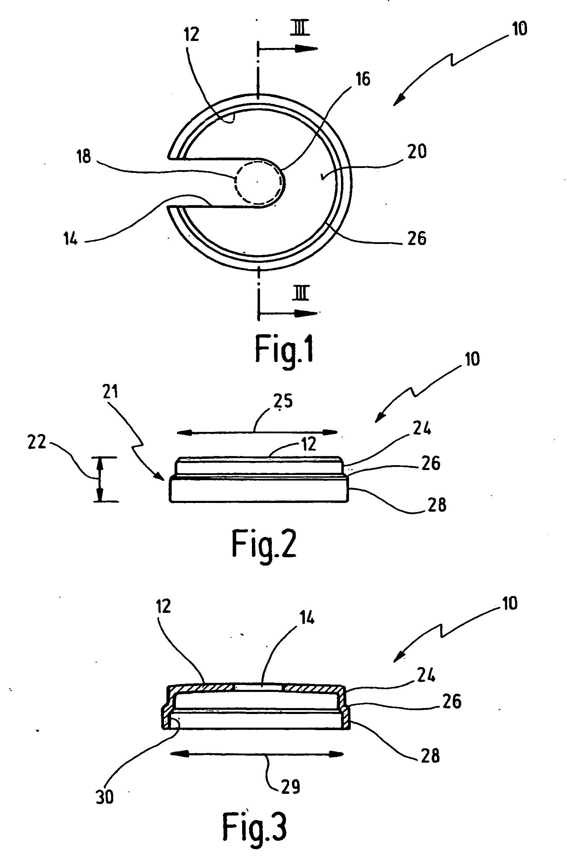

Ein in den Fig. 1 bis 4 dargestelltes Ausführungsbeispiel eines Abstandhalters ist in seiner Gesamtheit mit der Bezugsziffer 10 bezeichnet.

Der Abstandhalter 10 weist distalseitig grob die Form einer Scheibe 12 auf, in der eine seitlich öffnende Schlitzöffnung 14 ausgenommen ist. Die Schlitzöffnung 14 weist einen kreisförmigen Boden 16 auf, dessen Durchmesser und Lage so ist, dass der Abstandhalter 10 seitlich auf einen Schaft 18 eines später zu beschreibenden Instrumentes aufgeschoben werden kann, und zwar derart, dass dann der Schaft 18 etwa mittig in der geschlitzten Scheibe 12 zum Liegen kommt, wie das in Fig. 1 durch die gestrichelte Linie angedeutet ist.

Aus der Seitenansicht von Fig. 2 ist zu erkennen, dass sich von der Scheibe 12 ein erster Ringflansch 24 fort erstreckt, der über eine Schulter 26 in einen etwas durchmessergrößeren zweiten Ringflansch 28 übergeht. Diese bilden ein Abstandselement 21, das sich über einen Längenabschnitt 22 erstreckt.

Aus den Darstellungen von Fig. 2 und 3 ist zu erkennen, dass der Außendurchmesser 25 des ersten Ringflansches 24 in etwa dem lichten Innendurchmesser 29 des zweiten Ringflansches 28 entspricht. Dadurch ist es möglich, mehrere Abstandhalter 10 aufeinander zu setzen bzw. aufeinander zu stapeln, indem nämlich auf den ersten Ringflansch 24 eines Abstandhalters 10 der zweite Ringflansch 28 eines nächsten Abstandhalters 10' aufgesetzt wird.

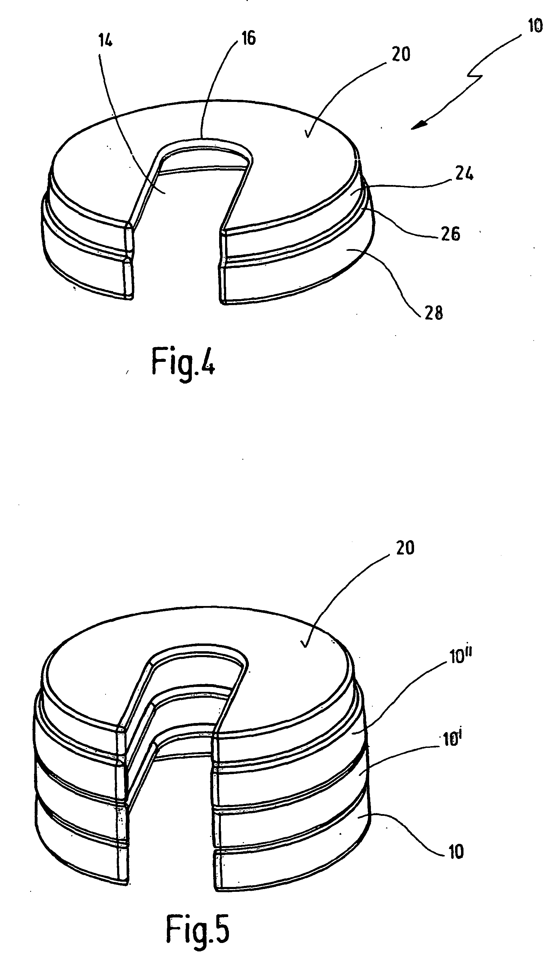

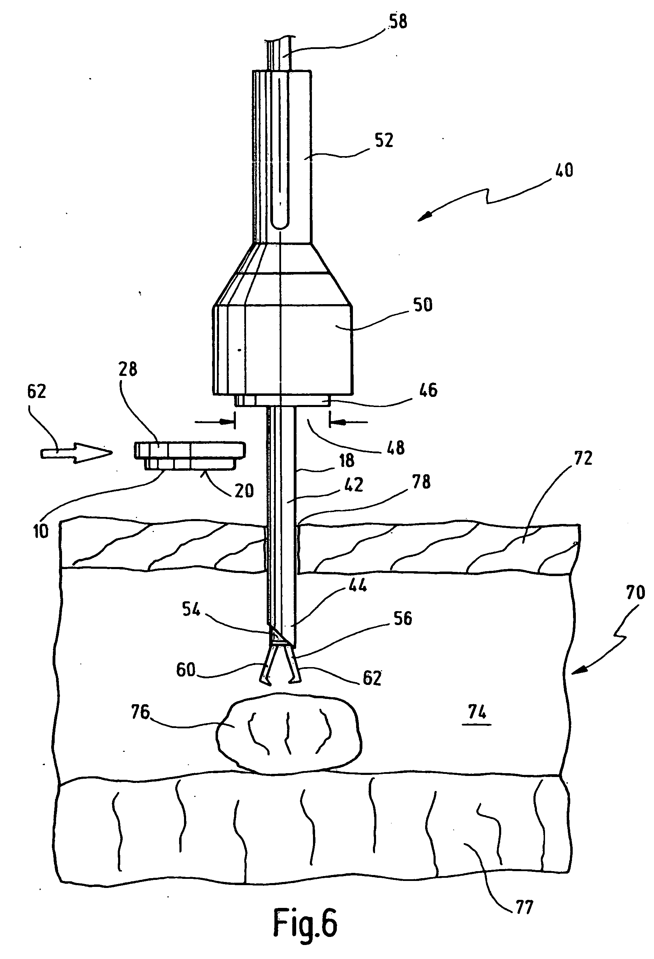

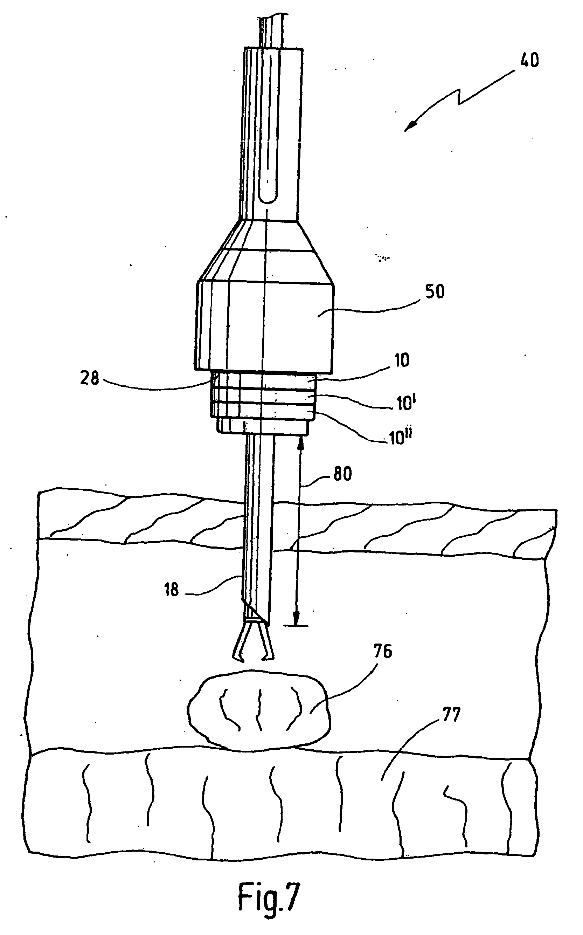

In Fig. 5 ist eine Situation dargestellt, bei der auf einen ersten Abstandhalter 10, wie er in Fig. 4 dargestellt ist, ein zweiter Abstandhalter 10' aufgesetzt ist und auf diesen wiederum ein dritter Abstandhalter 10" aufgesetzt ist. Die jeweils obere Fläche des obersten scheibenförmigen Körpers eines Abstandhalters bildet einen entsprechenden Anschlag 20, wie das nachfolgend in Zusammenhang mit Fig. 6 und 7 näher beschrieben wird.

Die Abstandhalter 10, 10', 10" können aus Kunststoffmaterial hergestellt werden, sie können aber auch aus metallischen Materialien hergestellt werden.

In Fig. 6 ist eine Situation dargestellt, bei der die Hülse 42 eines medizinischen Instruments 40 über eine Inzision 78 in einer Haut 72 eines menschlichen Körpers 70 in einen entsprechenden inneren Hohlraum 74 eingeschoben ist.

Das medizinische Instrument 40 ist als Morcellator ausgestaltet.

Das medizinische Instrument 40 weist eine Hülse 42 auf, die als ein Schaft 18 ausgebildet ist, wie er zuvor beschrieben ist.

Die Hülse 42 weist distalseitig eine angeschrägte Spitze 44 auf. Die Hülse 42 ist in einem Gehäuse 50 aufgenommen, dessen Durchmesser ein Mehrfaches des Durchmessers der Hülse 42 beträgt. Proximalseitig mündet das Gehäuse 50 in einen stabförmigen Griff 52, über den der Morcellator von Hand ergriffen werden kann.

Die Hülse 42 ist proximalseitig mit einem ringförmigen Vorsprung 46 versehen, über den die Hülse 42 am Gehäuse 50 montiert werden kann. Der Vorsprung 46 ist etwa scheibenförmig und weist einen Außendurchmesser 48 auf, der, wie das nachfolgend noch beschrieben wird, in etwa dem lichten Innendurchmesser 29 des zweiten Ringflansches 28 des Abstandhalters 10 entspricht.

Im Innern der Hülse 42 ist ein ebenfalls rohr- oder hülsenförmiges Schneidewerkzeug 54 aufgenommen, das distalseitig mit einer umlaufenden Schneide 56 versehen ist. Im Innern des hülsenförmigen Schneidewerkzeuges 54 ist noch eine Fasszange 58 durchgeschoben, deren Maulteile 60 und 62 distalseitig über das Schneidewerkzeug 54 hinausstehen und gerade gespreizt sind.

Die Maulteile 60 und 62 dienen dazu, einen Abschnitt eines abzutrennenden Gewebes 76 zu erfassen und zu halten, so dass dann dieser erfasste Gewebebereich, bei dann geschlossenen Maulteilen 60, 62 durch das Schneidewerkzeug 54 abgetrennt und ggf. durch den inneren Hohlraum sofort abgesaugt werden kann. Aus der Darstellung von Fig. 6 ist zu entnehmen, dass die Hülse 42 bzw. der Schaft 18 des Instrumentes 40 theoretisch so weit in den Körper 70 eingeschoben werden kann, bis das distalseitige Ende des Vorsprunges 46 an der Außenseite der Haut 72 zum Liegen kommt. In diesem Falle würde aber das Schneidewerkzeug 54 schon den eigentlich abzutrennenden Gewebebereich 76 durchdrungen haben und in einen darunter liegenden gesunden Gewebebereich 77 eingedrungen sein, der aber nicht abgetrennt werden soll.

Um nun die Einschubtiefe des Schaftes 18 bzw. der Hülse 42 des Instrumentes 40 zu begrenzen, können eine oder mehrere Abstandhalter 10, 10', 10" aufgeschoben werden. In Fig. 6 ist eine Situation dargestellt, wie der Abstandhalter 10 gerade seitlich an die Hülse 42 herangeführt wird, und zwar so weit, bis diese im Boden 16 der seitlichen Schlitzöffnung 14 zum Liegen kommt, wie das in Fig. 1 dargestellt ist. Je nach Ausgestaltung könnte nun der Abstandhalter 10 in dieser Position verbleiben, wenn beispielsweise der Abstandhalter 10 mit einer solchen Klemmkraft seitlich aufgeschoben werden muss, dass er axial kaum noch verschiebbar ist. Dies kann durch die entsprechende Wahl der Materialien bzw. der Bemaßung erreicht werden.

Würde der Operateur mit kräftigem Druck das Instrument 40 einschieben, kann nicht ausgeschlossen werden, dass der Abstandhalter 10 nach proximal längs der Hülse 42 verschoben wird. Das könnte maximal so weit gehen, bis der durchmessergrößere zweite Ringflansch 28 auf dem Vorsprung 46 zum Sitzen kommt.

Man kann daher vorsehen, dass dies von vornherein der Fall ist, also ein Abstandhalter 10 so angesetzt wird, bis er auf dem Vorsprung 46 zum Sitzen kommt.

Wird gewünscht, die Einschubtiefe um mehr um das Maß des Längenabschnitts 22 (s. Fig. 2) des Abstandhalters 10 zu begrenzen, kann ein Zusammenbau aus mehreren Abstandhaltern angesetzt werden, wie das in Fig. 7 dargestellt ist.

Hier ist nun ein Zusammenbau, wie er in Fig. 5 dargestellt ist, am Instrument 40 angesetzt, und zwar derart, dass der durchmessergrößere zweite Ringflansch 28 des untersten Abstandselementes 10 des Stapels auf dem Vorsprung 46 sitzt. Aus Fig. 7 ist zu entnehmen, dass die Einschubtiefe 80 entsprechend reduziert ist, nämlich um ein Maß aus der Summe der Höhe der drei zweiten Ringflansche 28 plus der Höhe des ersten Ringflansches 24 des obersten Abstandhalters 10" des Stapels. Durch diesen Zusammenbau der drei Abstandhalter 10, 10', 10" ist nun sichergestellt, dass das Instrument 40 gerade so weit eingeschoben werden kann, dass lediglich das abzutrennende Gewebe 76 abgetrennt werden kann und nicht das darunter gelegene Gewebe 77.

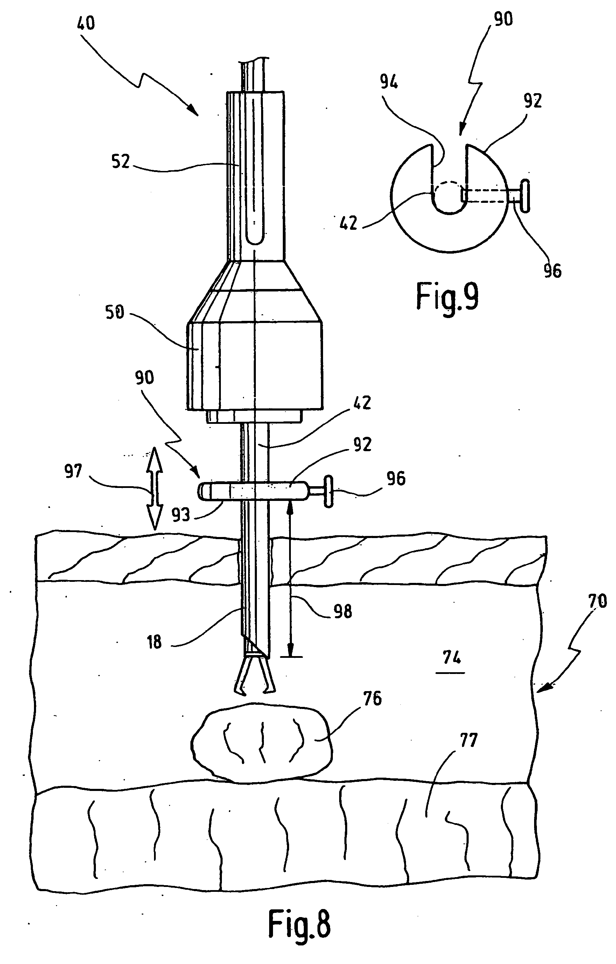

In den Fig. 8 und 9 ist eine weitere Ausführungsform eines Abstandhalters 90 dargestellt, der die Form einer Ringscheibe 92 aufweist, die seitlich mit einer Schlitzöffnung 94 versehen ist, deren lichte Breite in etwa dem Außendurchmesser der Hülse 42 des Instrumentes 40 entspricht, das identisch wie das zuvor dargestellte Instrument 40 ausgebildet ist, also ebenfalls einen Morcellator.

Im Körper der Ringscheibe 42 ist eine sich radial erstreckende Feststellschraube 96 aufgenommen, die in den Schlitz 94 hineingedreht werden kann und dadurch die Ringscheibe 52 in einer bestimmten Position am Schaft 42 feststellt.

Die Längsachse der Feststellschraube 96 ist etwa um 90° zur Längsachse der Schlitzöffnung 94 verdreht.

Durch Lösen der Feststellschraube 96 kann der Abstandhalter 90 in axialer Richtung entlang der Hülse 42 bewegt werden, wie das in Fig. 8 durch einen Doppelpfeil 97 dargestellt ist. Ist die gewünschte Position erreicht, wird der Abstandhalter 90 durch Festdrehen der Feststellschraube 96 an einer bestimmten Position fixiert. Es ist zu erkennen, dass die distalseitige Fläche der Ringscheibe 92 einen Anschlag 93 bildet. Dieser Anschlag 93 begrenzt dann die Einschubtiefe 98 der Hülse 42 in den Innenraum 74 des Körpers 70 wie zuvor beschrieben.

## Patentansprüche

1. Abstandhalter zur Begrenzung der Einschubtiefe (80, 98) eines Schaftes (18) eines medizinischen Instrumentes (40) in einen Körper (70), mit einem distalseitigen Anschlag (20, 93) zum Anlegen am Körper (70), mit einem sich über einen Längenabschnitt (22) des Schaftes (18) erstreckenden Abstandselement (21), wobei der Abstandhalter (10, 90) lösbar am medizinischen Instrument anbringbar ist, und mit einer Öffnung, über die der Abstandhalter (10, 90) am Schaft (18) anbringbar ist, **dadurch gekennzeichnet, dass** der Anschlag (20, 93) als scheibenartiger Körper ausgebildet ist, wobei die Öffnung als seitliche Schlitzöffnung (14; 94) im scheibenartigen Körper ausgebildet ist, über die der Abstandhalter (10, 10', 10", 90) seitlich an den Schaft (18) anlegbar ist, so dass der Schaft (18) etwa mittig in dem geschlitzten scheibenartigen Körper zum Liegen kommt.

2. Abstandhalter nach Anspruch 1, **dadurch gekennzeichnet, dass** mehrere Abstandhalter (10, 10', 10") aneinander koppelbar sind.

3. Abstandhalter nach Anspruch 1 oder 2, **dadurch gekennzeichnet, dass** das Abstandselement ein vom Anschlag (20) abstehenden ersten Ringflansch (24) aufweist, der über eine Schulter (26) in einen zweiten, durchmessergrößeren Ringflansch (28) übergeht.

4. Abstandhalter nach Anspruch 3, **dadurch gekennzeichnet, dass** der Außendurchmesser (25) des ersten Ringflansches (24) dem lichten Innendurchmesser (29) des zweiten Ringflansches (28) entspricht.

5. Abstandhalter nach Anspruch 3 oder 4, **dadurch gekennzeichnet, dass** der lichte Innendurchmesser (29) des zweiten Ringflansches (28) so gewählt ist, dass er vor einen entsprechenden Vorsprung (46) am Instrument (40) setzbar ist.

6. Abstandhalter nach einem der Ansprüche 1 bis 5, **dadurch gekennzeichnet, dass** das lösbare Anbringen durch eine Feststellschraube (96) erfolgt, über die ein an den Schaft (18) des Instruments (40) angelegter Abstandhalter (90) am Schaft (18) feststellbar ist.

7. Abstandhalter nach einem der Ansprüche 1 bis 6, **dadurch gekennzeichnet, dass** die Breite der Schlitzöffnung (14; 94) geringfügig geringer ist als der Außendurchmesser des Schaftes (18), so dass der Abstandhalter (10, 10', 10") im Klemmsitz an den Schaft (18) anlegbar ist.

## Claims

1. Spacer for limiting the depth of insertion (80, 98) of a shaft (18) of a medical instrument (40) into a body (70), with a distal abutment (20, 93) for bearing on the body (70), with a spacer element (21) extending along a length portion (22) of the shaft (18), wherein the spacer (10, 90) can be mounted releasably at the medical instrument, and having an aperture via which the spacer (10, 90) can be mounted on the shaft (18), **characterized in that** the abutment (20, 93) is designed as a disk-like body, wherein the aperture being designed as a lateral slit aperture (14; 94) within the disk-like body, via which the spacer (10, 10', 10", 90) can be fitted from a side onto the shaft (18) **in that** said shaft (18) be arranged approximately centrally in the slitted disk-like body.

2. Spacer of claim 1, **characterized in that** several spacers (10, 10', 10") can be coupled to one another.

3. Spacer of claims 1 or 2, **characterized in that** the spacer element has a first annual flange (24) which projects from the abutment (20) and which merges via a shoulder (26) into a second annular flange (28) of greater diameter.

4. Spacer of claim 3, **characterized in that** the external diameter (25) of the first annular flange (24) corresponds to the clear internal diameter (29) of the second annular flange (28).

5. Spacer of claims 3 or 4, **characterized in that** the clear internal diameter (29) of the second annular flange (28) is chosen such that it can be placed on a corresponding projection (46) on the instrument (40).

6. Spacer of anyone of claims 1 through 5, **characterized in that** the releasable mounting comprises a locking screw (96) via which a spacer (90) fitted on the shaft (80) of the instrument (40) can be locked on the shaft (18).

7. Spacer of anyone of claims 1 through 6, **characterized in that** the width of the slit aperture (14; 94) is slightly smaller than the external diameter of the shaft (18), so that the spacer (10, 10', 10") can be engaged with a press seat onto the shaft (18).

## Revendications

1. Entretoise pour limiter la profondeur d'enfoncement (80, 98) d'une tige (18) d'un instrument médical (40) dans un corps (70), avec une butée (20, 93) du côté distal pour s'appliquer contre le corps (70), avec un élément d'écartement (21) s'étendant sur une partie (22) de la longueur de la tige (18), sachant que l'entretoise (10, 90) peut être installée de manière amovible sur l'instrument médical, et avec une ouverture par laquelle l'entretoise (10, 90) peut être installée sur la tige (18), **caractérisée en ce que** la butée (20, 93) est réalisée sous forme de corps du genre disque, sachant que l'ouverture est réalisée sous la forme d'une ouverture en fente latérale (14 ; 94) dans le corps du genre disque, par laquelle l'entretoise (10, 10', 10", 90) peut être appliquée latéralement contre la tige (18), de sorte que la tige (18) vient se place environ centralement dans le corps fendu du genre disque.

2. Entretoise selon la revendication 1, **caractérisée en ce que** plusieurs entretoises (10, 10', 10") peuvent être accouplées les unes aux autres.

3. Entretoise selon la revendication 1 ou 2, **caractérisée en ce que** l'élément d'écartement présente un premier collet annulaire (24), qui fait saillie de la butée (20) et qui se raccorde par l'intermédiaire d'un épaulement (26) à un deuxième collet annulaire (28) de plus grand diamètre.

4. Entretoise selon la revendication 3, **caractérisée en ce que** le diamètre extérieur (25) du premier collet annulaire (24) correspond au diamètre intérieur de passage (29) du deuxième collet annulaire (28).

5. Entretoise selon la revendication 3 ou 4, **caractérisée en ce que** le diamètre intérieur de passage (29) du deuxième collet annulaire (28) est choisi de telle sorte qu'il peut être placé devant une saillie correspondante (46) prévue sur l'instrument (40).

6. Entretoise selon l'une des revendications 1 à 5, **caractérisée en ce que** l'installation amovible s'effectue par une vis de blocage (96) au moyen de laquelle une entretoise (90) appliquée contre la tige (18) de l'instrument (40) peut être bloquée sur la tige (18).

7. Entretoise selon l'une des revendications 1 à 6, **caractérisée en ce que** la largeur de l'ouverture en forme de fente (14 ; 94) est légèrement inférieure au diamètre extérieur de la tige (18), de sorte que l'entretoise (10, 10', 10") peut être appliquée en ajustement serré contre la tige (18).
